# EUROPEAN PATENT SPECIFICATION

(11) **EP 3 366 248 B1**
(45) Date of publication and mention of the grant of the patent: **15.05.2019**
(21) Application number: 18157893.1
(22) Date of filing: 21.02.2018
(51) Int. Cl.: A61B 18/14, A61B 90/90, A61B 90/92, A61B 5/00, A61M 25/00, A61B 18/00, A61B 34/20

(54) **CATHETER IDENTIFICATION SYSTEM AND METHOD**
SYSTEM UND VERFAHREN ZUR KATHETERIDENTIFIKATION
SYSTÈME ET PROCÉDÉ D'IDENTIFICATION DE CATHÉTER

(30) Priority: 22.02.2017 US 201715439490
(43) Date of publication of application: 29.08.2018
(73) Proprietor: Biosense Webster (Israel) Ltd., Yokneam 2066717 (IL)
(72) Inventor: DORON, Itai, 2066717 Yokneam (IL); COHEN, Assaf, 2066717 Yokneam (IL); COHEN-SACOMSKY, Hanna, 2066717 Yokneam (IL); GUTRAIMAN, Meidan, 2066717 Yokneam (IL)
(74) Representative: Carpmaels & Ransford LLP

(56) References cited:
- US-A- 5 489 275
- US-A1- 2011 218 546
- US-A1- 2014 005 475
- US-A1- 2016 242 666

## Description

### SUMMARY

In certain diagnostic and therapeutic techniques, including cardiac ablation, multiple catheters are inserted into chambers of the heart. During such procedures, a physician navigates the distal end of a catheter in a patient's body. The physician moves the distal end of the catheter by manipulating the proximal end of the catheter. One example of manipulating the proximal end includes using a handle fitted at the proximal end of the catheter.

Current catheter-based medical systems possess the ability to visualize a map of the distal end of the catheters within the heart on a display. Visualization is provided for multiple purposes, including for catheter manipulation. Accordingly, as a catheter is physically moved within the heart, the movement is visualized on the display for the physician. When multiple catheters are inserted into the patient's body, the physician can differentiate the catheters visualized on the display by selecting a specific color for each catheter on the display. The catheter sheaths are then highlighted on the display in the selected color for visualization during the procedure.

During medical procedures, the physician may be required to manipulate a single catheter while leaving the remaining inserted catheters in place. Current catheter-based medical systems, however, lack a proper identification system that is in sync with both the catheter handle and the display. The physician has no indication of which catheter handle corresponds to which catheter visualized on the display screen. Thus, in order to determine which catheter handle corresponds to a specific catheter on the display screen, the physician must physically move the catheter within the patient's body and simultaneously observe the visualization on the display to identify which catheter is moving. A system for visually identifying catheters for both catheter handles and display visualizations would therefore be useful to increase efficiency of medical procedures and prevent complications.

US2016242666 and US5489275 refer to documents relevant for the present invention.

A system and method for identifying a catheter inserted in a patient's body, as claimed hereinafter, is provided.

### BRIEF DESCRIPTION OF THE DRAWINGS

FIG. 1 is a schematic, pictorial illustration of a catheter identification system as it is used by a physician.
FIG. 2 is a schematic representation of a catheter visualization image on a display of a catheter identification system.
FIG. 3 is a pictorial illustration of a catheter handle as it relates to a catheter identification system.
FIG. 4 is a block diagram of electrical components of a catheter identification system.

### DETAILED DESCRIPTION OF THE PREFERRED EMBODIMENTS

The present invention is defined by the appended claims. A system and method for catheter identification may be configured such that the color of a catheter depicted on a catheter visualization image on a display of a console reflects the color of an LED or electronic display on the catheter handle. This system may permit more efficient cardiac medical procedures by helping identify the catheter within a patient's body. In addition, a configurable color identification on both the image in the display of the console and the catheter handle may help decrease unnecessary movement of catheters by a physician to identify which catheter is the desired catheter to manipulate. Thus, this configuration may prevent catheter identification confusion and harm to the catheter or patient. This configuration may also allow other procedural data to be displayed on the catheter handle, including force and temperature information, the type of catheter being used, duration of ablation, and various alerts.

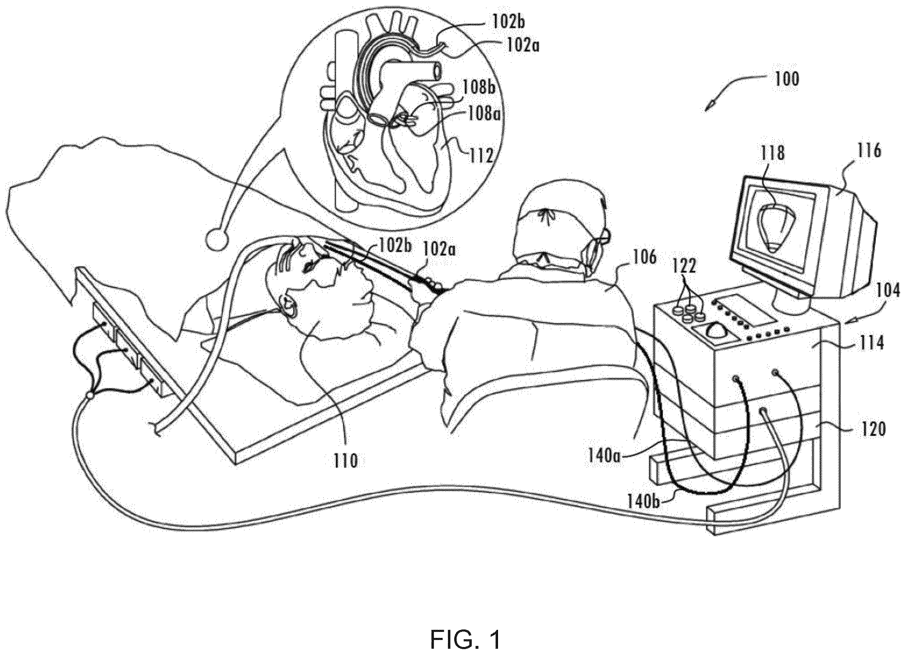

FIG. 1 is a schematic, pictorial illustration of a catheter identification system as it is used by a physician. The catheter identification system 100 may be based, for example, on the CARTO® systems, produced by Biosense Webster Inc. (Diamond Bar, Calif.). These systems may utilize electromagnetic technology to create real-time three-dimensional (3D) maps of a patient's cardiac structures. The catheter identification system 100 comprises invasive probes in the form of a catheter 102a and a catheter 102b, as well as a control console 104. The console 104 includes a display 116, a processor 114, a memory 120, and one or more input devices 122. In other embodiments, the system 100 may comprise additional catheters.

In the present embodiment, the catheter 102a and the catheter 102b may be used in mapping or ablating endocardial tissue. In other embodiments, the catheters 102a and 102b and the catheter identification system 100 may be used to perform other of therapeutic or diagnostic procedures. Catheters for mapping and/or ablation typically carry one or more magnetic position sensors for generating signals that are used to determine position coordinates of a distal portion of the catheter. Magnetic field generators are thus driven to generate magnetic fields in the vicinity of the patient. The field generators comprise coils, which are placed below the patient's torso at known positions external to the patient. The coils generate magnetic fields that are sensed by the magnetic position sensors carried in the catheter. Electrical signals are generated by the sensors and are then passed to a signal processor via leads that extend through the catheter. During a catheter ablation procedure, once the catheter reaches the heart, radiofrequency (RF) energy is delivered to specific areas of the heart wall to produce a small lesion, to block faulty electrical impulses that can cause heart rhythm disorders. The 3D map image that is generated by the system aids a physician in steering the catheter to areas in the heart where RF energy needs to be administered.

During ablation or other therapeutic or diagnostic procedures, a physician may insert several catheters into a patient's body. The insertion of multiple catheters makes it difficult to distinguish an individual catheter handle and the corresponding catheter within the patient's body for manipulating a specific catheter. Current catheter systems do not include an identification system for a desired catheter handle among multiple catheters inserted within a patient's body, and the corresponding desired catheter on the 3D map image. Instead, in order to differentiate a desired catheter handle to manipulate a desired catheter, the physician must move the catheter handle to observe the catheter movement on the generated 3D map image. By having a physician move other catheters in order to identify the desired catheter within the patient's body to manipulate, inefficiency of the procedure is increased and identification confusion resulting in harm to the catheter or patient is likely to occur.

For example, a physician may select one of multiple catheters on a display depicting a 3D map image of the catheters in a patient's cardiac structures to be colored blue. The physician may select this color to differentiate the specific catheter on the image from the other catheters. The physician, however, has no way of identifying which catheter in the patient's body is the blue catheter displayed on the screen. The physician thus has to move each catheter within the patient's body until the blue catheter on the display is observed to move with respect to the catheter being moved by the physician. Unnecessary movement of the catheters within the patient's body to identify the desired catheter may decrease efficiency of the medical procedure, may damage the catheter, and may even be harmful to the patient.

Returning to FIG. 1, in the present embodiment, an operator 106, such as a physician, inserts the catheter 102a and the catheter 102b through the vascular system of a patient 110 so that a distal end 108a of catheter 102a and a distal end 108b of catheter 102b enter a chamber of the patient's heart 112. The operator advances the catheters so that a tip of the distal end 108a and a tip of the distal end 108b engage endocardial tissue at desired locations. The catheter 102a is connected by a suitable cable 140a at its proximal end to the console 104. In addition, the catheter 102b is connected by a suitable cable 140b at its proximal end to the console 104. The console 104 uses a position sensing technique to determine position coordinates of the distal end 108a of the catheter 102a and the distal end 108b of the catheter 102b inside the heart 112.

The console 104 uses any position sensing technique, including magnetic position sensing. The signal processor 114 processes the position sensing data in order to determine the position coordinates of the distal end 108a and the distal end 108b, including both location and orientation coordinates. The processor 114 may comprise a general-purpose computer, with suitable front end and interface circuits for receiving signals from the catheter 102a and the catheter 102b and controlling the other components of the console 104. The processor 114 may be programmed in software to carry out the functions described herein. The software may be downloaded to the console 104 in electronic form, over a network, for example, or it may be provided on tangible media, such as optical, magnetic or electronic memory media. In other embodiments, some or all of the functions of the processor 114 may be carried out by dedicated or programmable digital hardware components.

Based on the signals received from the catheter 102a and the catheter 102b, as well as from other components of the catheter identification system 100, the processor 114 drives the display 116 to give the operator 106 visual feedback through an image 118. The image 118 depicts a 3D map of the distal end 108a of the catheter 102a and the distal end 108b of the catheter 102b in the patient's body, as well as status information and guidance regarding the procedure that is in progress. The image 118 provides accurate visualization of multiple catheters in the patient's heart 112 and pinpoints the exact location and orientation of the catheter 102a and the catheter 102b. The processor 114 may store data representing the image 118 in the memory 120.

The operator 106 may manipulate the image 118 using the input device 122 on the console 104. The operator 106 may thus use the input device 122 to select configurable colors from a range of different colors to uniquely identify both the catheter 102a and the catheter 102b on the image 118. The configurable color input data for the catheter 102a and the catheter 102b on the image 118 is then communicated to the processor 114. The processor 114 then communicates the color input to the catheter 102a and the catheter 102b to display the colors selected by the operator 106 in the image 118 on the body of the respective catheter.

For example, if the operator 106 wishes the catheter 102a to have a red sheath color and the catheter 102b to have a blue sheath color on the image 118, the operator 106 selects the colors using the input device 122 on the console 104 to input the colors into the identification system 100. Thus, the catheter 102a is depicted on the image 118 with a red sheath color, while the catheter 102b is depicted on the image 118 with a blue sheath color. The configurable color input is then communicated to the processor 114. The processor then communicates both to the catheter 102a inserted within the patient's body to display a red color on the catheter 102a, and to the catheter 102b inserted within the patient's body to display a blue color on the catheter 102b. Thus, the colors depicted on the handle of the catheter 102a and the handle of the catheter 102b, respectively, reflect the colors of the catheter sheath of the catheter 102a and the catheter sheath of the catheter 102b on the image 118, respectively.

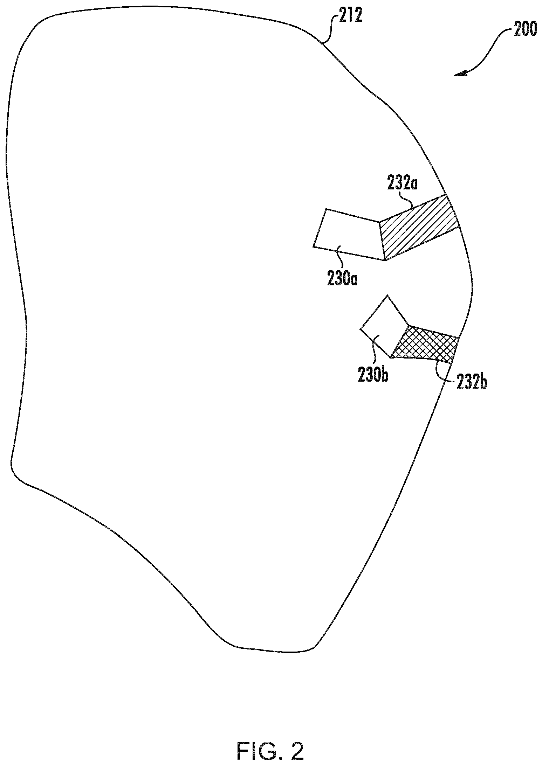

FIG. 2 is a schematic representation of a catheter visualization image on a display of a catheter identification system. The image 200 includes an icon 230a corresponding to the distal end 208a of the catheter 102a, as depicted in FIG. 1, and an icon 230b corresponding to the distal end 208b of the catheter 102b, as depicted in FIG. 1. In other embodiments, more than two icons corresponding to more than two catheters in the patient's body are depicted in the image 200.

In the present embodiment, the image 200 is illustrated on a display (not depicted), as an aid in visualizing the distal end of the catheters within the heart. The image includes a 3D graphical map of an inner surface 212 of the heart chamber in which the distal end of the catheters are located. Surface 212 may be fully reconstructed, as shown in FIG. 2, or only partially reconstructed. The position of the icon 230a and the icon 230b relative to the surface 212 gives an indication of the location of the actual distal ends of the catheters in the heart chamber.

At least a portion 232a of the icon 230a and a portion 232b of the icon 230b may display a uniquely identifiable color (represented in the figure by hatching) on the image 200, as input by an operator (not depicted). Accordingly, as the operator manipulates the catheter on the display, it will reflect or display that selected color. This may be manifested in a solid color, colored outline, a color indicator, or the like. For example, the catheter 102a, as depicted in FIG. 1, may be marked a green color on the image 200 to distinguish the catheter 102a on the image 200. In the present embodiment, the portion of the icons displaying the uniquely identifiable color may reflect the sheath of each catheter. In other embodiments, the portion may reflect any other part of the catheter.

Current catheter systems, however, do not include an identification system for a catheter handle among multiple catheters inserted within a patient's body. A physician must instead move the catheter handle to observe the catheter movement on the generated 3D image. By having a physician move other catheters in order to identify the desired catheter within the patient's body to manipulate, inefficiency of the procedure is increased and harm to the catheter or patient is likely to occur. Returning to FIG. 2, the identification of a desired catheter 102a, as depicted in FIG. 1, in both the image 200 and the catheter 102a inserted within the patient's body through a configurable color may allow for a more efficient catheter identification process, and may reduce identification confusion and catheter or patient harm.

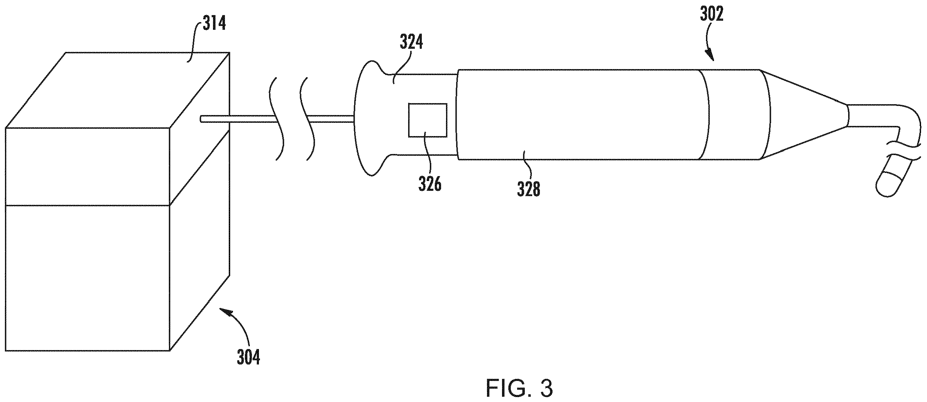

FIG. 3 is a pictorial illustration of a catheter handle as it relates to a catheter identification system. The catheter identification system 300 includes an example catheter 302. The catheter 302 includes a handle 324. The handle 324 includes a microprocessor 326 and a handle display 328. The microprocessor 326 controls the handle display 328 and provides an identifiable state to the handle display 328 including, but not limited to, color or graphical information to reflect the identification color input in the console 304.

Thus, when a configurable color is input in the console 304 to identify the catheter 302, the input color selection for the catheter 302, i.e. the configurable color input, is communicated to the processor 314. In the present embodiment, the processor 314 is located in the console 304. The processor 314 may comprise a general-purpose computer, with suitable front end and interface circuits for receiving signals from the catheter 302 and the console 304, and for controlling both the color displayed on the catheter 302 and other components of the console 304. Based on the input color selection for the catheter 302 that is input into the console 304, the processor 314 communicates the configurable color input to the microprocessor 326 on the handle 324 of the catheter 302. The microprocessor 326 communicates with the handle display 328 by sending a signal to display the particular configurable color input in the console 304.

The handle display 328 may comprise a light emitting diode (LED) or electronic display. In the present embodiment, the handle display 328 includes a multicolored LED and textual display. The LED allows the system 300 to display a color, e.g. red, blue, etc., on the catheter handle 324 in order to uniquely identify the catheter 302. In other embodiments, the handle display 328 may include a graphic display on the catheter handle 324, or may include various combinations of the multicolored LED, textual, and graphic displays. The textual or graphic display may be used to communicate additional information including force or temperature parameters, type of catheter used, duration of ablation, and various alerts.

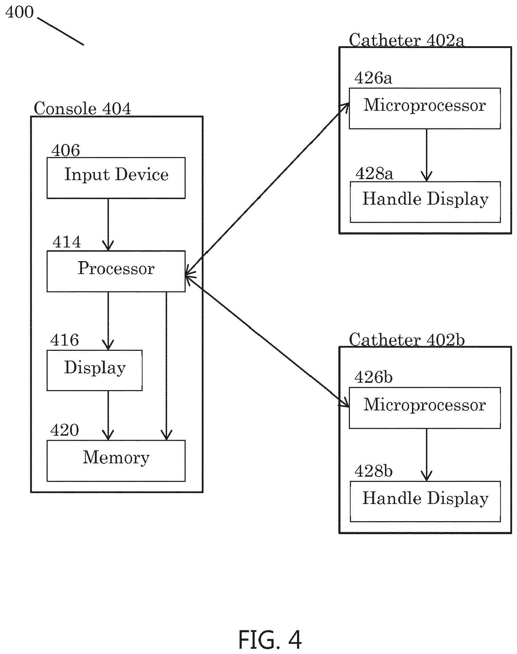

FIG. 4 is a block diagram of electrical components of a catheter identification system 400. In the present embodiment, the catheter identification system 400 comprises a console 404 and two catheters: catheter 402a and catheter 402b. In other embodiments, the catheter identification system may comprise more than two catheters. The console includes an input device 406, a processor 414, a display 416, and a memory 420. The input device 406, the display 416, and the memory 420 are coupled to the processor 414. The catheter 402a includes a microprocessor 426a and a handle display 428a. The catheter 402b similarly includes a microprocessor 426b and a handle display 428b. The processor 414 is coupled to the catheter 402a via microprocessor 426a; and is also coupled to the catheter 402b via microprocessor 426b.

In the present embodiment, a configurable color is input in the input device 406 to uniquely identify catheter 402a. The input device 406 then communicates the configurable color input to the signal processor 414 in the console 404. The processor 414 may comprise a general-purpose computer, with suitable front end and interface circuits for receiving signals from the input device 406, the catheter 402a, and the catheter 402b. The processor 414 also controls the display 416 and the microprocessor 426a of the catheter 402a and the microprocessor 426b of the catheter 402b to depict the selected colors for catheter identification.

Based on the signals received from the input device in the console 404 regarding the configurable color input, the processor 414 displays the color input on the display 416 in the console 404 by depicting an image of the catheter 402a having the selected color. Accordingly, as the operator manipulates the identified catheter on the display 416, the catheter 402a on the display 416 will reflect or depict that selected color. This may be manifested in a color outline, a solid color, a color indicator or the like. The processor 414 may then store the configurable color input data in the memory 420. The processor 414 may also communicate with the display 416 to store the displayed image in memory 420. The processor 414 also sends the configurable color input data to the microprocessor 426a on the catheter 402a. The microprocessor 426a then programs the handle display 428a on the catheter 402a to display the input color from the console. Thus, the color of the handle display 428a of the catheter 402a reflects the same color of the catheter 402a in the image on the display 416.

A different configurable color may be input in the input device 406 of the console 404 in a similar manner for the catheter 402b. The input device 406 communicates the configurable color input to the signal processor 414. The processor 414 then displays the color input on the display 416 in the console 404 containing an image of the catheter 402b. Thus, the selected color will be reflected as color outline, a solid color, a color indicator, or the like as the operator manipulates the catheter depicted on the display 416. The processor 414 may then store the configurable color input data in the memory 420 and may also communicate with the display 416 to store the image in memory 420. The processor 414 also sends the configurable color input data to the microprocessor 426b on the catheter 402b. The microprocessor 426b programs the handle display 428b on the catheter 402b to display the input color from the console.

For example, a configurable color input of blue to identify catheter 402a and a configurable color input of red for catheter 402b may be input in the input device 406. The configurable color input data is then sent to the processor 414, which communicates with the display 416 to depict catheter 426a as blue and catheter 426b as red on the display 416 in the console 404. The processor 414 then transmits the color input data to microprocessor 426a, which programs a handle display 428a to display a blue color for the catheter 402a. The processor 414 also transmits the color input data to microprocessor 426b, which programs the handle display 428b to display a red color for the catheter 402b.

As a result, the catheter 402a and the catheter 402b are each uniquely identified by a different configurable color, and this color is reflected in the image of the catheters on the display 416. The configurable colors input for the catheter 402a and the catheter 402b, and thus the colors depicted for catheter 402a and catheter 402b on the display 416 and the handle display 428a and the handle display 428b, may be changed at any time by selecting a color from a range of colors on the input device 406.

## Claims

1. A system (100) for identifying a catheter inserted in a patient's body, comprising:
a catheter (302) comprising:
a handle (324), comprising:
an invasive probe having a proximal end connected to the handle and a distal tip, and
a magnetic position sensing assembly located within the invasive probe; and
a console (104) comprising:
an input device (122),
a display screen (116) configured to depict an image of the catheter inside the patient's body, and
a processor (114) configured to communicate between the input device, the display screen, and the catheter;
wherein the processor receives a configurable color input from the input device, depicts the configurable color on the image (118) of the catheter on the display screen, and displays the configurable color on the handle (324) of the corresponding catheter within the patient's body;
wherein the handle further comprises:
a microprocessor (326) configured to receive the configurable color input, and
a handle display (328) configured to display the selected color.

2. The system of claim 1, wherein the processor (114) is configured to use the magnetic position sensing assembly to sense the position of the distal tip relative to the distal end (208a, 208b) of the invasive probe and to display an icon (230a, 230b) on the display screen that represents the catheter.

3. The system according to claim 1, wherein the handle display comprises:
a light emitting diode configured to display a color out of a range of colors, and
a textual display.

4. The system according to claim 1, wherein the handle display further comprises a graphic display.

5. The system according to claim 1, wherein the handle display is further configured to display information including force and temperature data, the type of catheter being used, ablation duration, and alerts.

6. The system according to claim 1, wherein the console further comprises a memory configured to store the configurable color input and the image of the catheter on the display.

7. A method for identifying a catheter (302) inserted in a patient's body, comprising:
inputting a configurable color for the catheter into an input device (122) by selecting the configurable color from a range of colors,
acquiring the configurable color input by a processor,
displaying the configurable color on an image (118) of the catheter on a display screen (116), and
displaying the configurable color on a handle (324) of the catheter, wherein said displaying the configurable color on the handle further comprises:
communicating the configurable color input to a microprocessor (326) on the handle by the processor, and
displaying the configurable color on a handle display (328) by the microprocessor.

8. The method according to claim 7, wherein said displaying the configurable color on a handle display by the microprocessor further comprises programming a light emitting diode on the handle display to display the selected color.

9. The method according to claim 7, further comprising displaying information including force and temperature data, the type of catheter being used, ablation duration, and alerts on a textual display on the handle display.

10. The method according to claim 7, further comprising storing the configurable color input and the image of the catheter on the display screen in a memory(420).

## Patentansprüche

1. System (100) zum Identifizieren eines in den Körper eines Patienten eingesetzten Katheters, umfassend:
einen Katheter (302), umfassend:
einen Handgriff (324), umfassend:
eine invasive Sonde, die ein mit dem Handgriff verbundenes proximales Ende und eine distale Spitze aufweist, und
eine magnetische Positionserfassungsanordnung, die innerhalb der invasiven Sonde angeordnet ist; und
eine Konsole (104), umfassend:
eine Eingabevorrichtung (122),
einen Anzeigebildschirm (116), der konfiguriert ist, um ein Bild des Katheters in dem Körper des Patienten darzustellen, und
einen Prozessor (114), der konfiguriert ist, um zwischen der Eingabevorrichtung, dem Anzeigebildschirm und dem Katheter zu kommunizieren;
wobei der Prozessor eine konfigurierbare Farbeingabe von der Eingabevorrichtung empfängt, die konfigurierbare Farbe auf dem Bild (118) des Katheters auf dem Anzeigebildschirm darstellt und die konfigurierbare Farbe auf dem Handgriff (324) des entsprechenden Katheters innerhalb des Körpers des Patienten anzeigt;
wobei der Handgriff ferner umfasst:
einen Mikroprozessor (326), der konfiguriert ist, um die konfigurierbare Farbeingabe zu empfangen, und
eine Handgriffanzeige (328), die konfiguriert ist, um die ausgewählte Farbe anzuzeigen.

2. System nach Anspruch 1, wobei der Prozessor (114) konfiguriert ist, um die magnetische Positionserfassungsanordnung zu verwenden, um die Position der distalen Spitze relativ zu dem distalen Ende (208a, 208b) der invasiven Sonde zu erfassen und ein Symbol (230a, 230b) auf dem Anzeigebildschirm anzuzeigen, das den Katheter darstellt.

3. System nach Anspruch 1, wobei die Handgriffanzeige umfasst:
eine lichtemittierende Diode, die konfiguriert ist, um eine Farbe aus einer Reihe von Farben anzuzeigen, und
eine Textanzeige.

4. System nach Anspruch 1, wobei die Handgriffanzeige ferner eine grafische Anzeige umfasst.

5. System nach Anspruch 1, wobei die Handgriffanzeige ferner konfiguriert ist, um Informationen einschließlich Kraft- und Temperaturdaten, die Art des verwendeten Katheters, die Ablationsdauer und Warnungen anzuzeigen.

6. System nach Anspruch 1, wobei die Konsole ferner einen Speicher umfasst, der konfiguriert ist, um die konfigurierbare Farbeingabe und das Bild des Katheters auf der Anzeige zu speichern.

7. Verfahren zum Identifizieren eines in den Körper eines Patienten eingesetzten Katheters (302), umfassend:
Eingeben einer konfigurierbaren Farbe für den Katheter in eine Eingabevorrichtung (122) durch Auswählen der konfigurierbaren Farbe aus einer Reihe von Farben,
Übernehmen der konfigurierbaren Farbeingabe durch einen Prozessor,
Anzeigen der konfigurierbaren Farbe auf einem Bild (118) des Katheters auf einem Anzeigebildschirm (116), und
Anzeigen der konfigurierbaren Farbe an einem Handgriff (324) des Katheters, wobei das Anzeigen der konfigurierbaren Farbe an dem Handgriff ferner umfasst:
Übermitteln der konfigurierbaren Farbeingabe an einen Mikroprozessor (326) an dem Handgriff durch den Prozessor, und
Anzeigen der konfigurierbaren Farbe auf einer Handgriffanzeige (328) durch den Mikroprozessor.

8. Verfahren nach Anspruch 7, wobei das Anzeigen der konfigurierbaren Farbe auf einer Handgriffanzeige durch den Mikroprozessor ferner das Programmieren einer lichtemittierenden Diode auf der Handgriffanzeige zum Anzeigen der ausgewählten Farbe umfasst.

9. Verfahren nach Anspruch 7, ferner umfassend das Anzeigen von Informationen einschließlich Kraft- und Temperaturdaten, die Art des verwendeten Katheters, die Ablationsdauer und Warnungen auf einer Textanzeige auf der Handgriffanzeige.

10. Verfahren nach Anspruch 7, ferner umfassend das Speichern der konfigurierbaren Farbeingabe und des Bildes des Katheters auf dem Anzeigebildschirm in einem Speicher (420).

## Revendications

1. Système (100) pour identifier un cathéter inséré dans le corps d'un patient, comprenant :
un cathéter (302) comprenant :
une poignée (324) comprenant :
une sonde invasive ayant une extrémité proximale raccordée à la poignée et une pointe distale, et
un ensemble de détection de position magnétique situé à l'intérieur de la sonde invasive ; et
une console (104) comprenant :
un dispositif d'entrée (122),
un écran de visualisation (116) configuré pour représenter une image du cathéter à l'intérieur du corps du patient, et
un processeur (114) configuré pour communiquer entre le dispositif d'entrée, l'écran de visualisation et le cathéter ;
dans lequel le processeur reçoit une entrée de couleur configurable en provenance du dispositif d'entrée, représente la couleur configurable sur l'image (118) du cathéter sur l'écran de visualisation et affiche la couleur configurable sur la poignée (324) du cathéter correspondant à l'intérieur du corps du patient ;
dans lequel la poignée comprend en outre :
un microprocesseur (326) configuré pour recevoir l'entrée de couleur configurable ; et
un dispositif d'affichage de poignée (328) configuré pour afficher la couleur sélectionnée.

2. Système selon la revendication 1, dans lequel le processeur (114) est configuré pour utiliser l'ensemble de détection de position magnétique pour détecter la position de la pointe distale par rapport à l'extrémité distale (208a, 208b) de la sonde invasive et pour afficher une icône (230a, 230b) sur l'écran de visualisation qui représente le cathéter.

3. Système selon la revendication 1, dans lequel le dispositif d'affichage de poignée comprend :
une diode électroluminescente configurée pour afficher une couleur parmi une gamme de couleurs, et
un dispositif d'affichage de texte.

4. Système selon la revendication 1, dans lequel le dispositif d'affichage de poignée comprend en outre un dispositif d'affichage graphique.

5. Système selon la revendication 1, dans lequel le dispositif d'affichage de poignée est en outre configuré pour afficher des informations comportant des données de force et de température, le type de cathéter qui est utilisé, la durée d'ablation et des alertes.

6. Système selon la revendication 1, dans lequel la console comprend en outre une mémoire configurée pour stocker l'entrée de couleur configurable et l'image du cathéter sur le dispositif d'affichage.

7. Procédé pour identifier un cathéter (302) inséré dans le corps d'un patient, consistant :
à entrer une couleur configurable pour le cathéter dans un dispositif d'entrée (122) en sélectionnant la couleur configurable parmi une gamme de couleurs,
à acquérir l'entrée de couleur configurable au moyen d'un processeur,
à afficher la couleur configurable sur une image (118) du cathéter sur un écran de visualisation (116) et
à afficher la couleur configurable sur une poignée (324) du cathéter, dans lequel ledit affichage de la couleur configurable sur la poignée consiste en outre :
à communiquer l'entrée de couleur configurable à un microprocesseur (326) sur la poignée au moyen du processeur, et
à afficher la couleur configurable sur un dispositif d'affichage de poignée (328) au moyen du microprocesseur.

8. Procédé selon la revendication 7, dans lequel ledit affichage de la couleur configurable sur un dispositif d'affichage de poignée au moyen du microprocesseur consiste en outre à programmer une diode électroluminescente sur le dispositif d'affichage de poignée pour afficher la couleur sélectionnée.

9. Procédé selon la revendication 7, consistant en outre à afficher des informations comportant des données de force et de température, le type de cathéter qui est utilisé, la durée d'ablation et des alertes sur un dispositif d'affichage de texte sur le dispositif d'affichage de poignée.

10. Procédé selon la revendication 7, consistant en outre à stocker l'entrée de couleur configurable et l'image du cathéter sur l'écran de visualisation dans une mémoire (420).
